# EUROPÄISCHE PATENTSCHRIFT

(11) **EP 0 575 897 B1**
(45) Veröffentlichungstag und Bekanntmachung des Hinweises auf die Patenterteilung: **06.03.1996**
(21) Anmeldenummer: 93109741.4
(22) Anmeldetag: 18.06.1993
(51) Int. Cl.: B01J 23/88

(54) **Multimetalloxidmassen**
Multimetal oxide masses
Masses d'oxydes multimétalliques

(30) Priorität: 25.06.1992 DE 4220859
(43) Veröffentlichungstag der Anmeldung: 29.12.1993
(73) Patentinhaber: BASF Aktiengesellschaft, D-67063 Ludwigshafen (DE)
(72) Erfinder: Neumann, Hans-Peter, Dr., D-6800 Mannheim 31 (DE); Martan, Hans, Dr., D-6710 Frankenthal (DE); Petersen, Hermann, Dr., D-6718 Gruenstadt (DE); Doerflinger, Walter, D-7524 Oestringen (DE)

(56) Entgegenhaltungen:
- EP-A- 0 000 835
- FR-A- 2 534 904

## Beschreibung

Die vorliegende Erfindung betrifft Massen der allgemeinen Formel I

[X¹ₐX²_{b}Oₓ]ₚ [X³_{c}X⁴_{d}X⁵ₑX⁶_{f}X⁷_{g}X²ₕO_{y}]_{q} (I),

in der die Variablen folgende Bedeutung haben:
- X¹: Wismut, Tellur, Antimon, Zinn und/oder Kupfer,
- X²: Molybdän und/oder Wolfram,
- X³: ein Alkalimetall, Thallium und/oder Samarium,
- X⁴: ein Erdalkalimetall, Nickel, Kobalt, Kupfer, Mangan, Zink, Zinn, Cadmium und/oder Quecksilber,
- X⁵: Eisen, Chrom, Cer und/oder Vanadium,
- X⁶: Phosphor, Arsen, Bor und/oder Antimon,
- X⁷: ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
- a: 0,01 bis 8,
- b: 0,1 bis 30,
- c: 0 bis 4,
- d: 0 bis 20,
- e: 0 bis 20,
- f: 0 bis 6,
- g: 0 bis 15,
- h: 8 bis 16,
- x,y: Zahlen, die durch die Wertigkeit und Häufigkeit der von Sauerstoff verschiedenen Elemente in I bestimmt werden und
- p,q: Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen chemischen Zusammensetzung abgegrenzte, Bereiche der chemischen Zusammensetzung X¹ₐX²_{b}Oₓ, deren Größtdurchmesser (längste durch den Schwerpunkt des Bereichs gehende Verbindungsstrecke zweier auf der Oberfläche (Grenzfläche) des Bereichs befindlicher Punkte) 1 bis 25 µm, vorzugsweise 1 bis 20 µm und besonders bevorzugt 5 bis 15 µm beträgt.

Außerdem betrifft vorliegende Erfindung ein Verfahren zur Herstellung dieser Massen sowie deren Verwendung (die experimentelle Ermittlung der Größtdurchmesser gestattet z.B. die Methode der energiedispersiven Röntgenanalyse (EDXS), z.B. an einer Elektronenstrahl-Mikrosonde JEOL JCXA/733).

Massen der allgemeinen Formel I sind aus der EP-A 835 bekannt.

Hinsichtlich der Herstellung dieser Massen empfiehlt die EP-A 835 das Mischoxid X¹ₐX²_{b}Oₓ in Abwesenheit der übrigen Konstituenten der Massen I vorzubilden, es nach seiner Vorbildung mit Quellen der übrigen Konstituenten der Massen I zu vermischen und das Gemisch nach Trocknung zu calcinieren.

Im einzelnen kann die Herstellung so erfolgen, daß man zur Vorbildung von X¹ₐX²_{b}Oₓ aus lösliche Verbindungen der Elemente X¹, X² enthaltenden Lösungen in geeigneter Weise fällt, das gefällte Produkt nach Trocknen und Calcinieren in einer Kugelmühle mahlt und dann das Pulver mit einer wäßrigen Mischung, die die Quellen der übrigen Konstituenten der Massen I enthält, vermischt, das Gemisch trocknet und calciniert oder die Quellen der übrigen Konstituenten der das durch geeignetes Fällen vorgebildete Produkt enthaltenden Aufschlämmung unmittelbar zusetzt und die dabei resultierende Mischung trocknet und calciniert.

Weiterhin ist aus der EP-A 835 bekannt, die Massen der allgemeinen Formel I als Katalysatoren für gasphasenkatalytische Oxidationen organischer Verbindungen einzusetzen. Nachteilig an den in der EP-A 835 offenbarten Massen der allgemeinen Formel I ist jedoch, daß sie bei Anwendung in gasphasenkatalytischen Oxidationen organischer Verbindungen sowohl hinsichtlich Aktivität als auch Selektivität nicht voll zu befriedigen vermögen.

Aus der DE-C 33 38 380 sind katalytisch aktive Massen der allgemeinen Formel II

Biₐ,W_{b},Fe_{c},Mo_{d},Y¹ₑ,Y²_{f},Y³_{g},Y⁴ₕ,Oₓ, (II),

in der die Variablen folgende Bedeutung haben: 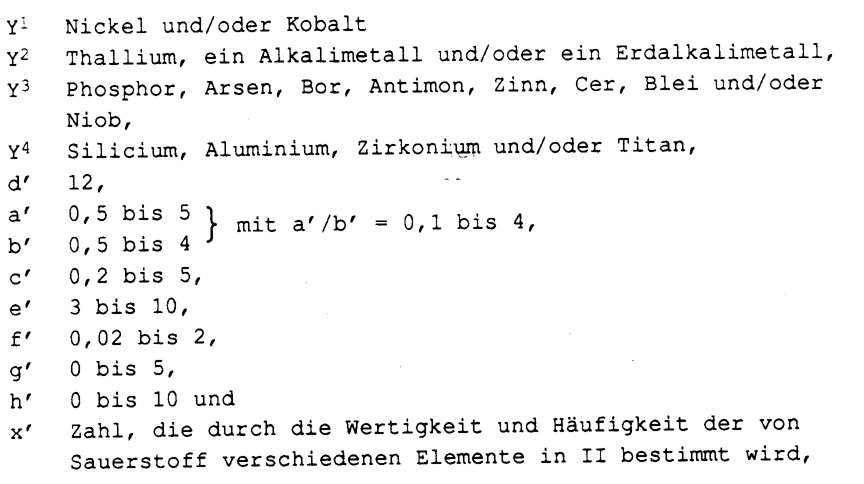
bekannt, die dadurch erhalten werden, daß man zunächst eine Wismut- und eine Wolframverbindung in wäßrigem Medium vermischt, das wäßrige Gemisch trocknet, die resultierende Masse bei 600 bis 900°C calciniert und anschließend so pulverisiert, daß die Korngröße weniger als 152 µm beträgt, sowie das dabei erhaltene Pulver mit einer wäßrigen Lösung der Quellen der übrigen Konstituenten der Masse II versetzt, die resultierende Mischung einengt, formt und calciniert.

Weiterhin ist aus der DE-C 33 38 380 bekannt, daß die Massen II als Katalysatoren für die gasphasenkatalytisch-oxidative Herstellung ungesättigter Aldehyde geeignet sind.

Nachteilig an den in der DE-C 33 38 380 offenbarten Massen II ist jedoch, daß sie bei Anwendung zur gasphasenkatalytisch-oxidativen Herstellung von ungesättigten Aldehyden sowohl hinsichtlich Aktivität als auch Selektivität nicht voll zu befriedigen vermögen.

Aufgabe der vorliegenden Erfindung war daher, Massen der allgemeinen Formel I zur Verfügung zu stellen, die als Katalysatoren für gasphasenkatalytische Oxidationen organischer Verbindungen, insbesondere zur gasphasenkatalytischoxidativen Herstellung ungesättigter Aldehyde sowie ungesättigter Carbonsäuren, hinsichtlich Aktivität und Selektivität eine erhöhte Wirksamkeit aufweisen.

Demgemäß wurden die eingangs definierten Massen gefunden.

Solche Massen I sind bevorzugt, die wenigstens eines der Elemente Eisen, Chrom, Cer, Vanadium oder ein anderes seltenes Erdmetall mit einem von O verschiedenen stöchiometrischen Koeffizienten enthalten, der mit Vorteil ≧ 0,01 beträgt.

Besonders vorteilhafte erfindungsgemäße Massen I sind dabei solche, in denen X¹ Wismut ist. Unter diesen werden wiederum jene bevorzugt, die der allgemeinen Formel III

[Bi_{a''}Z²_{b''}O_{x''}]_{p''}[Z²₁₂Z³_{c''}Z⁴_{d''}Fe_{e''}Z⁵_{f''}Z⁶_{g''}Z⁷_{h''}O_{y''}]_{q''} (III)

, in der die Variablen folgende Bedeutung haben:
- Z²: Molybdän und/oder Wolfram,
- Z³: Nickel und/oder Kobalt,
- Z⁴: Thallium, ein Alkalimetall und/oder ein Erdalkalimetall,
- Z⁵: Phosphor, Arsen, Bor, Antimon, Zinn, Cer und/oder Blei,
- Z⁶: Silicium, Aluminium, Titan und/oder Zirkonium,
- Z⁷: Kupfer, Silber und/oder Gold,
- a'': 0,1 bis 1,
- b'': 0,2 bis 2,
- c'': 3 bis 10,
- d'': 0,02 bis 2,
- e'': 0,01 bis 5, vorzugsweise 0,1 bis 3,
- f'': 0 bis 5,
- g'': 0 bis 10,
- h'': 0 bis 1 und
- x'',y'': Zahlen, die durch die Wertigkeit und Häufigkeit der von Sauerstoff verschiedenen Elemente in III bestimmt werden,
- p'',q'': Zahlen, deren Verhältnis p''/q'' 0,1 bis 5, vorzugsweise 0,5 bis 2 beträgt,
entsprechen, wobei diejenigen Massen III ganz besonders bevorzugt werden, in denen Z²_{b''} = (Wolfram)_{b''} und Z²₁₂ = (Molybdän)₁₂ ist.

Ferner ist es von Vorteil, wenn wenigstens 50 mol-% (bevorzugt 100 mol-%) des gesamten Anteils [X¹ₐX²_{b}Oₓ]ₚ ([Bi_{a''}Z²_{b''}O_{x''}]_{p''}) der erfindungsgemäßen Massen I (Massen III) in den erfindungsgemäßen Massen in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung X¹ₐX²_{b}Oₓ (Bi_{a''}Z²_{b''}O_{x''}) vorliegen, deren Größtdurchmesser teilweise im Bereich von 1 bis 25 µm (vorzugsweise 1 bis 20 und besonders bevorzugt 5 bis 15 µm) liegt.

Je größer der prozentuale Anteil der Anzahl der verschiedenen Größtdurchmesser ist (bezogen auf die Gesamtzahl der vorhandenen Bereiche), der einen Wert im Bereich 1 bis 25 µm (vorzugsweise 1 bis 20 und besonders bevorzugt 5 bis 15 µm) aufweist, desto vorteilhafter erweisen sich die erfindungsgemäßen Massen.

D.h. vorzugsweise liegt wenigstens die Hälfte der Größtdurchmesser im Intervall 1 bis 25 µm (mit Vorteil im Intervall 1 bis 20 und besonders bevorzugt im Intervall 5 bis 15 µm) und ganz besonders bevorzugt liegen alle Größtdurchmesser in diesem Intervall.

Die erfindungsgemäßen Massen I sind in besonders geeigneter Weise z.B. dadurch erhältlich, daß man zunächst in an sich bekannter Weise (vgl. EP-A 835 und DE-C 33 38 380) ein calciniertes Mischoxid X¹ₐX²_{b}Oₓ vorbildet, (z.B. wasserlösliche Salze von X¹ wie Nitrate, Carbonate, Hydroxide oder Acetate mit X²-Säuren oder deren Ammoniumsalzen in Wasser mischen, die Mischung trocknen (vorzugsweise sprühtrocknen) und die getrocknete Masse calcinieren), es zerkleinert (z.B. in der Kugelmühle oder durch Strahlmahlen) und aus dem dabei erhältlichen, in der Regel aus im wesentlichen kugelförmigen Partikeln bestehenden, Pulver die Kornklasse mit einem im für die Masse I gewünschten Größtdurchmesserbereich liegenden Korngrößtdurchmesser durch in an sich bekannter Weise durchzuführendes Klassieren (z.B. Naß- oder Trockensiebung) abtrennt und vorzugsweise mit, bezogen auf die Masse dieser abgetrennten Kornklasse, vorzugsweise 0,1 bis 3 Gew.-%, feinteiligem SiO₂ (der zahlenmittlere Korngrößtdurchmesser der üblicherweise im wesentlichen kugelförmigen SiO₂-Partikel beträgt zweckmäßigerweise 10 bis 50 nm) vermischt und so eine Ausgangsmasse 1 herstellt.

Mit Vorteil handelt es sich bei dem vorgebildeten calcinierten Mischoxid um ein solches der Stöchiometrie BiZ²O₆, Bi₂Z²₂O₉ und/oder Bi₂Z²₃O₁₂, unter denen das Bi₂Z²₂O₉ bevorzugt ist, insbesondere wenn Z² = Wolfram.

Die Calcinierungstemperatur beträgt zweckmäßig 400 bis 900°C, vorzugsweise 600 bis 900°C. Üblicherweise erfolgt die Calcinierung im Luftstrom. Die Calcinierungsdauer erstreckt sich in der Regel auf wenige Stunden.

Von den übrigen Bestandteilen der gewünschten erfindungsgemäßen Masse wird ausgehend von in an sich bekannter Weise geeigneten Quellen (vgl. EP-A 835 und DE-C 33 38 380) ein möglichst inniges, vorzugsweise feinteiliges, Trockengemisch hergestellt (z.B. wasserlösliche Salze wie Halogenide, Nitrate, Acetate, Carbonate oder Hydroxide in einer wäßrigen Lösung vereinen und anschließend die wäßrige Lösung Sprühtrocknen oder nicht wasserlösliche Salze, z.B. Oxide, in wäßrigem Medium suspendieren und anschließend die Suspension sprühtrocknen), das hier als Ausgangsmasse 2 bezeichnet wird. Wesentlich ist nur, daß es sich bei den Bestandteilen der Ausgangsmasse 2 entweder bereits um Oxide handelt, oder um solche Verbindungen, die durch Erhitzen, gegebenenfalls in Anwesenheit von Sauerstoff, in Oxide überführbar sind.

Anschließend werden die Ausgangsmasse 1 und die Ausgangsmasse 2 im gewünschten Mengenverhältnis miteinander vermischt, vorzugsweise durch Pressen verdichtet, und danach (normalerweise im Luftstrom) zweckmäßig bei Temperaturen von 400 bis 600°C, mehrere Stunden calciniert. In einer weniger bevorzugten Ausführungsform kann das vorgebildete calcinierte Mischoxid X¹ₐX²_{b}Oₓ mit Quellen der übrigen Bestandteile der gewünschten erfindungsgemäßen Masse auch in flüssigem, vorzugsweise wäßrigem, Medium innig vermischt werden. Dieses Gemisch wird anschließend zu einem innigen Trockengemisch getrocknet und sodann, wie oben beschrieben, geformt oder ungeformt, calciniert. Dabei können die Quellen der übrigen Konstituenten in diesem flüssigen Medium gelöst und/oder suspendiert vorliegen, wohingegen das vorgebildete calcinierte Mischoxid in diesem flüssigen Medium im wesentlichen unlöslich sein sollte, d.h. suspendiert vorliegen muß.

Im Falle von Vollkatalysatoren erfolgt das Verpressen unmittelbar zur gewünschten Katalysatorgeometrie, wobei als solche Hohlzylinder mit einem Außendurchmesser und einer Länge von 2 bis 10 mm und einer Wandstärke von 1 bis 3 mm bevorzugt werden. Die aktiven erfindungsgemäßen Massen I können nach dem Calcinieren aber auch zerkleinert und auf inerte Träger zur Herstellung von Trägerkatalysatoren aufgebracht werden. Das Aufbringen kann auch bereits vor der abschließenden Calcinierung erfolgen. In diesem Fall erfolgt das Aufbringen vorzugsweise gemäß der EP-B 293 859. Selbstverständlich können die erfindungsgemäßen Massen auch in Pulverform eingesetzt werden.

Die erfindungsgemäßen Massen eignen sich insbesondere als Katalysatoren mit erhöhter Aktivität und Selektivität für gasphasenkatalytische Oxidationen organischer Verbindungen wie niederer (3 bis 6 C-Atome) Alkane, Alkanole, Alkanale, Alkene und Alkenale zu olefinisch ungesättigten Aldehyden und/oder Carbonsäuren, sowie den entsprechenden Nitrilen (Ammonoxidation, vor allem von Propen zu Acrylnitril und von 2-Methylpropen bzw. tert.-Butanol zu Methacrylnitril). Sie eignen sich aber auch zur oxidativen Dehydrierung organischer Verbindungen.

Insbesondere eignen sich die erfindungsgemäßen Massen, besonders die Massen III, zur gasphasenkatalytisch-oxidativen Herstellung von Acrolein, Acrylsäure, Methacrolein und Methacrylsäure, wobei als Ausgangsverbindungen vorzugsweise Propen oder 2-Methylpropen bzw. tert.-Butanol eingesetzt werden. Besonders vorteilhaft erwiesen sich die erfindungsgemäßen Massen dabei als Katalysatoren zur Herstellung von Acrolein und Methacrolein.

Als Oxidationsmittel wird in an sich bekannter Weise Sauerstoff, zweckmäßigerweise mit inerten Gasen verdünnt, eingesetzt. Als inerte Gase kommen z.B. N₂ oder Wasserdampf in Betracht. Reaktionstemperatur und Reaktionsdruck sind dem Fachmann bekannt.

### Beispiele

### a) Herstellung von Ausgangsmassen 1

50 kg einer Lösung von Bi(NO₃)₃ in wäßriger Salpetersäure (11 Gew.-% Bi, 6,4 Gew.-% HNO₃, jeweils auf die Lösung bezogen) wurde mit 6,7 kg H₂WO₄ versetzt und 1 h bei 50°C gerührt.

Die erhaltene Suspension wurde sprühgetrocknet und 2 h bei 750°C calciniert. Das so erhaltene vorgebildete calcinierte Mischoxid (Bi₂W₂O₉ mit geringer WO₃-Verunreinigung) wurde gemahlen und in folgende Korngrößtdurchmesser(d)fraktionen klassiert:
- VF1:: 0,1 µm < d ≦ 1 µm
- F2:: 1 µm < d ≦ 5 µm
- F3:: 5 µm < d ≦ 10 µm
- F4:: 10 µm < d ≦ 15 µm
- F5:: 15 µm < d ≦ 20 µm
- F6:: 20 µm < d ≦ 25 µm
- VF7:: 30 µm < d ≦ 50 µm
- VF8:: 90 µm < d ≦ 120 µm
Anschließend wurden alle Fraktionen mit 1 % ihres Gewichtes an feinteiligem (zahlenmittlerer Größtdurchmesser 28 nm) SiO₂ vermengt.

### b) Herstellung einer Ausgangsmasse 2

Eine Lösung von 85,5 kg Ammoniummolybdat in 240 l Wasser wurde mit einer Lösung die 11,9 kg Kobalt(II)nitrat und 5,7 kg Eisen(III)nitrat in 80 l Wasser gelöst enthielt sowie mit 7,8 kg einer 20 % ihres Gewichtes kolloidales SiO₂ enthaltenden wäßrigen Mischung und 377 g einer 48 Gew.-% KOH enthaltenden wäßrigen Lösung versetzt. Anschließend wurde 3 h gerührt und die dabei resultierende wäßrige Suspension sprühgetrocknet.

### c) Herstellung von Massen I

Die verschiedenen feinteiliges SiO₂ enthaltenden Fraktionen VF1 bis VF8 wurden jeweils für sich mit der Ausgangsmasse 2 in der für eine Masse I der Zusammensetzung

[Bi₂W₂O₉]_{0,5}Mo₁₂Co₅Fe_{2,5}Si_{1,6}K_{0,05}Oₓ

erforderlichen Menge vermischt und zu Hohlzylindern mit 5 mm Länge, 5 mm Außendurchmesser und 1,5 mm Wandstärke verpreßt und anschließend während 6 h bei 470°C im Luftstrom calciniert. Untersuchungen der resultierenden Masse I mittels energiedispersiver Röntgenanalyse (EDXS) an einer Elektronenstrahl-Mikrosonde JEOL JCXA/733 ergaben, daß die resultierenden Massen I dreidimensional ausgedehnte, von ihrer lokalen Umgebung aufgrund ihrer von ihrer lokalen Umgebung verschiedenen chemischen Zusammensetzung abgegrenzte, Bereiche der chemischen Zusammensetzung Bi₂W₂O₉ aufwiesen, deren Größtdurchmesser im wesentlichen den Korngrößtdurchmessern der zu ihrer Herstellung verwendeten Kornfraktion VF1 bis VF8 entsprachen.

### d) Gasphasenkatalytische Oxidation von Propen

Ein jeweils mit 1200 ml der jeweiligen Masse I aus c) gefülltes Reaktionsrohr (V2A, 2 mm Wandstärke, 25 mm Innendurchmesser, Salzbadtemperierung) wurde mit 2400 Nl/h einer Gasmischung der Zusammensetzung 5 Vol.-% Propen, 9 Vol.-% Sauerstoff und 86 Vol.-% Stickstoff beschickt.

Die Salzbadtemperatur wurde in allen Fällen so eingestellt, daß bei einfachem Durchgang eine Propenumwandlung von ca. 98 mol-% resultierte.

Die diesbezüglich als Funktion der zur Herstellung der eingesetzten Masse I in Form der jeweiligen Ausgangsmasse 1 verwendeten Fraktionen VF1 bis VF8 erforderlichen Salzbadtemperaturen (Maß für die Aktivität der eingesetzten Masse I) sowie die resultierenden Selektivitäten (bezogen auf die Summe von gebildetem Acrolein und Acrylsäure) zeigt die Tabelle.

**Tabelle**

| | Propenumwandlung [mol-%] | Salzbadtemperatur [°C] | Selektivität [mol-%] |
|---|---|---|---|
| VF1 | 98 | 350 | 95,8 |
| F2 | 97,9 | 338 | 96,8 |
| F3 | 98,1 | 330 | 96,3 |
| F4 | 97,9 | 322 | 95,7 |
| F5 | 98,0 | 322 | 95,0 |
| F6 | 97,9 | 337 | 94,3 |
| VF7 | 98,0 | 355 | 93,8 |
| VF8 | 98,0 | 382 | 90,8 |

Wie ersichtlich, durchlaufen sowohl die Aktivität als auch die Selektivität ein ausgeprägtes Maximum, wobei bei Kombination beider Größen solche Massen I als besonders vorteilhaft ausgewiesen werden , bei denen die Größtdurchmesser der in ihnen enthaltenen chemisch abgegrenzten Bereiche der Zusammensetzung Bi₂W₂O₉ ausschließlich im Bereich von 1 bis 25 µm liegen.

Nach einer Betriebszeit von 500 h wurden die jeweils eingesetzten Massen I nochmals einer energiedispersiven Röntgenanalyse unterworfen. Dabei ergab sich, daß die chemisch abgegrenzten Bereiche der Zusammensetzung Bi₂W₂O₉ hinsichtlich ihrer Größtdurchmesser im wesentlichen unverändert vorlagen.

## Patentansprüche

1. Massen der allgemeinen Formel I
[X¹ₐX²_{b}Oₓ]ₚ [X³_{c}X⁴_{d}X⁵ₑX⁶_{f}X⁷_{g}X²ₕO_{y}]_{q} (I),
in der die Variablen folgende Bedeutung haben:
X¹ Wismut, Tellur, Antimon, Zinn und/oder Kupfer,
X² Molybdän und/oder Wolfram,
X³ ein Alkalimetall, Thallium und/oder Samarium,
X⁴ ein Erdalkalimetall, Nickel, Kobalt, Kupfer, Mangan, Zink, Zinn, Cadmium und/oder Quecksilber,
X⁵ Eisen, Chrom, Cer und/oder Vanadium,
X⁶ Phosphor, Arsen, Bor und/oder Antimon,
X⁷ ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
a 0,01 bis 8,
b 0,1 bis 30,
c 0 bis 4,
d 0 bis 20,
e 0 bis 20,
f 0 bis 6,
g 0 bis 15,
h 8 bis 16,
x,y Zahlen, die durch die Wertigkeit und Häufigkeit der von Sauerstoff verschiedenen Elementen in I bestimmt werden und
p,q Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen chemischen Zusammensetzung abgegrenzte, Bereiche der chemischen Zusammensetzung X¹ₐX²_{b}Oₓ, deren Größtdurchmesser 1 bis 25 µm beträgt.

2. Massen nach Anspruch 1, enthaltend den gesamten Anteil [X¹ₐX²_{b}Oₓ]ₚ in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung X¹ₐX²_{b}Oₓ.

3. Massen nach Anspruch 1 oder 2, in denen die Anzahl der lokal abgegrenzten Bereiche der Zusammensetzung X¹ₐX²_{b}Oₓ, die einen Größtdurchmesser im Bereich von 1 bis 25 µm aufweisen, bezogen auf die Gesamtzahl der enthaltenen lokal abgegrenzten Bereiche der Zusammensetzung X¹ₐX²_{b}Oₓ, wenigstens 50 % beträgt.

4. Massen nach den Ansprüchen 1 bis 3, in denen alle vorhandenen lokal abgegrenzten Bereiche der Zusammensetzung X¹ₐX²_{b}Oₓ einen Größtdurchmesser im Bereich von 1 bis 25 µm aufweisen.

5. Massen nach den Ansprüchen 1 bis 4, in denen X¹ Wismut ist.

6. Massen nach den Ansprüchen 1 bis 5, in denen X¹ₐX²_{b}Oₓ identisch mit Bi₂W₂O₉ ist.

7. Massen der allgemeinen Formel I, dadurch erhältlich, daß man zunächst ein feinteiliges Pulver aus calciniertem Mischoxid X¹ₐX²_{b}Oₓ, dessen Korngrößtdurchmesser im Bereich von 1 bis 25 µm liegen, als Ausgangsmasse 1 vorbildet, aus Quellen der übrigen Bestandteile der gewünschten Masse I eine feinteilige trockene Ausgangsmasse 2, die diese Quellen in inniger Mischung und in bereits oxidischer und/oder in durch Calcinieren in oxidische Form überführbarer Form enthält, herstellt, die Ausgangsmasse 1 und die Ausgangsmasse 2 im gewünschten Mengenverhältnis miteinander vermischt und das Gemisch abschließend calciniert.

8. Verfahren zur Herstellung von Massen gemäß den Ansprüchen 1 bis 7, dadurch gekennzeichnet, daß man zunächst ein feinteiliges Pulver aus calciniertem Mischoxid X¹ₐX²_{b}Oₓ, dessen Korngrößtdurchmesser im Bereich von 1 bis 25 µm liegen, als Ausgangsmasse 1 vorbildet, aus Quellen der übrigen Bestandteile der gewünschten Masse I eine feinteilige trockene Ausgangsmasse 2, die diese Quellen in inniger Mischung und in bereits oxidischer und/oder in durch Calcinieren in oxidische Form überführbarer Form enthält, herstellt, die Ausgangsmasse 1 und die Ausgangsmasse 2 im gewünschten Mengenverhältnis miteinander vermischt und das Gemisch abschließend calciniert.

9. Verwendung von Massen gemäß den Ansprüchen 1 bis 7 als Katalysatoren für gasphasenkatalytische Oxidationen organischer Verbindungen.

10. Verfahren zur Herstellung von α,β-monoethylenisch ungesättigten Aldehyden und/oder Carbonsäuren die 3 bis 6 C-Atome aufweisen durch gasphasenkatalytische Oxidation eines dieselbe Anzahl an C-Atomen aufweisenden Alkans, Alkanols, Alkens und/oder Alkenals, dadurch gekennzeichnet, daß als Katalysator eine Masse gemäß den Ansprüchen 1 bis 7 verwendet wird.

## Claims

1. A composition of the formula I
[X¹ₐX²_{b}Oₓ]ₚ [X³_{c}X⁴_{d}X⁵ₑX⁶_{f}X⁷_{g}X²ₕO_{y}]_{q} (I)
where
X¹ is bismuth, tellurium, antimony, tin and/or copper,
X² is molybdenum and/or tungsten,
X³ is an alkali metal, thallium and/or samarium,
X⁴ is an alkaline earth metal, nickel, cobalt, copper, manganese, zinc, tin, cadmium and/or mercury,
X⁵ is iron, chromium, cerium and/or vanadium,
X⁶ is phosphorus, arsenic, boron and/or antimony,
X⁷ is a rare-earth metal, titanium, zirconium, niobium, tantalum, rhenium, ruthenium, rhodium, silver, gold, aluminum, gallium, indium, silicon, germanium, lead, thorium and/or uranium,
a is from 0.01 to 8,
b is from 0.1 to 30,
c is from 0 to 4,
d is from 0 to 20,
e is from 0 to 20,
f is from 0 to 6,
g is from 0 to 15,
h is from 8 to 16,
x and y are numbers determined by the valency and frequency of the elements in I other than oxygen, and p and q are numbers whose ratio p/q is from 0.1 to 10, containing three-dimensional regions with a chemical composition X¹ₐX²_{b}Oₓ which are delimited from their local environment due to their chemical composition which is different from their local environment, and whose maximum diameter is from 1 to 25 µm.

2. A composition as claimed in claim 1, containing all the [X¹ₐX²_{b}Oₓ]ₚ in the form of three-dimensional regions with the chemical composition X¹ₐX²_{b}Oₓ which are delimited from their local environment due to their chemical composition which is different from their local environment.

3. A composition as claimed in claim 1, in which the number of locally delimited regions with the composition X¹ₐX²_{b}Oₓ which have a maximum diameter in the range from 1 to 25 µm, is, based on the total number of locally delimited regions with the composition X¹ₐX²_{b}Oₓ, of at least 50%.

4. A composition as claimed in claim 1, in which all the locally delimited regions with the composition X¹ₐX²_{b}Oₓ have a maximum diameter in the range from 1 to 25 µm.

5. A composition as claimed in claim 1, where X¹ is bismuth.

6. A composition as claimed in claim 1, where X¹ₐX²_{b}Oₓ is identical with Bi₂W₂O₉.

7. A composition of the formula I, obtainable by first preparing a finely divided powder from calcined mixed oxide X¹ₐX²_{b}Oₓ whose maximum particle diameter is in the range from 1 to 25 µm, as starting composition 1, preparing a finely divided dry starting composition 2 from sources of the other constituents of the desired composition I, the starting composition 2 containing these sources in an intimate mixture and in a form which is already oxidic and/or can be converted into oxidic form by calcination, mixing the starting composition 1 and the starting composition 2 in the desired mixing ratio, and subsequently calcining the mixture.

8. A process for the preparation of a composition as claimed in claim 1, which comprises first preparing a finely divided powder from calcined mixed oxide X¹ₐX²_{b}Oₓ whose maximum particle diameter is in the range from 1 to 25 µm, as starting composition 1, preparing a finely divided dry starting composition 2 from sources of the other constituents of the desired composition I, the starting composition 2 containing these sources in an intimate mixture and in a form which is already oxidic and/or can be converted into oxidic form by calcination, mixing the starting composition 1 and the starting composition 2 in the desired mixing ratio, and subsequently calcining the mixture.

9. A method of using a composition as claimed in claim 1 as a catalyst for the gas-phase catalytic oxidation of organic compounds.

10. A process for the preparation of α,β-monoethylenically unsaturated aldehydes and/or carboxylic acids having 3 to 6 carbon atoms by gas-phase catalytic oxidation of an alkane, alkanol, alkene and/or alkenal having the same number of carbon atoms, which comprises using, as catalyst, a composition as claimed in claim 1.

## Revendications

1. Masses de la formule générale I
[X¹ₐX²_{b}Oₓ]ₚ [X³_{c}X⁴_{d}X⁵ₑX⁶_{f}X⁷_{g}X²ₕO_{y}]_{q} (I),
dans laquelle les diverses variables ont les significations qui suivent :
X¹ bismuth, tellure, antimoine, étain et/ou cuivre,
X² molybdène et/ou tungstène,
X³ un métal alcalin, thallium et/ou samarium,
X⁴ un métal alcalino-terreux, nickel, cobalt, cuivre, manganèse, zinc, étain, cadmium et/ou mercure,
X⁵ fer, chrome, cérium et/ou vanadium,
X⁶ phosphore, arsenic, bore et/ou antimoine,
X⁷ un métal des terres rares, titane, zirconium, niobium, tantale, rhénium, ruthénium, rhodium, argent, or, aluminium, gallium, indium, silicium, germanium, plomb, thorium et/ou uranium,
a 0,01 à 8,
b 0,1 à 30,
c 0 à 4,
d 0 à 20,
e 0 à 20,
f 0 à 6,
g 0 à 15,
h 8 à 16,
x,y des nombres qui sont déterminés par la valence et la fréquence des éléments qui diffèrent de l'oxygène dans I et
p,q des nombres dont le rapport p/q varie de 0,1 à 10, contenant des domaines de la composition chimique X¹ₐX²_{b}Oₓ, étendus dans les trois dimensions, limités par leur environnement local sur base de leur composition chimique différant de leur environnement local, dont le plus grand diamètre varie de 1 à 25 µm.

2. Masses suivant la revendication 1, contenant la fraction [X¹ₐX²_{b}Oₓ]ₚ totale sous forme de domaines de la composition chimique X¹ₐX²_{b}Oₓ étendus dans les trois dimensions, limité par leur environnement local sur base de leur composition chimique différant de leur environnement local.

3. Masses suivant la revendication 1 ou 2, dans lesquelles le nombre de domaines localement limités de la composition X¹ₐX²_{b}Oₓ, qui présentent un plus grand diamètre dans la plage de 1 à 25 µm, atteint au moins 50% par rapport au nombre total des domaines localement limités contenus de la composition X¹ₐX²_{b}Oₓ.

4. Masses suivant les revendications 1 à 3, dans lesquelles tous les domaines localement limités présents de la composition X¹ₐX²_{b}Oₓ présentent un plus grand diamètre qui varie de 1 à 25 µm.

5. Masses suivant les revendications 1 à 4, dans lesquelles X¹ représente le bismuth.

6. Masses suivant les revendications 1 à 5, dans lesquelles X¹ₐX²_{b}Oₓ est identique à Bi₂W₂O₉.

7. Masses de la formule I, que l'on peut obtenir par le fait que l'on prépare d'abord, à titre de masses de départ 1, une poudre finement divisée d'un oxyde mixte calciné X¹ₐX²_{b}Oₓ dont les plus grands diamètres des grains se situent dans la plage de 1 à 25 µm, à partir de sources des autres constituants de la masse souhaitée I, on prépare une masse de départ 2 sèche et finement divisée, qui contient ces sources en mélange intime et déjà sous forme oxydique et/ou convertible en forme oxydique par calcination, on mélange mutuellement la masse de départ 1 et la masse de départ 2 en le rapport quantitatif souhaité et on calcine ensuite le mélange.

8. Procédé de préparation de masses suivant les revendications 1 à 7, caractérisé en ce que l'on prépare d'abord, à titre de masses de départ 1, une poudre finement divisée d'un oxyde mixte calciné X¹ₐX²_{b}Oₓ dont les plus grands diamètres des grains se situent dans la plage de 1 à 25 µm, à partir de sources des autres constituants de la masse souhaitée I, on prépare une masse de départ 2 sèche et finement divisée, qui contient ces sources en mélange intime et déjà sous forme oxydique et/ou convertible en forme oxydique par calcination, on mélange mutuellement la masse de départ 1 et la masse de départ 2 en le rapport quantitatif souhaité et on calcine ensuite le mélange.

9. Utilisation de masses suivant les revendications 1 à 7 à titre de catalyseurs pour des oxydations catalytiques en phases gazeuses de composés organiques.

10. Procédé de préparation d'acides carboxyliques et/ou d'aldéhydes α,β-monoéthyléniquement insaturés, qui comportent de 3 à 6 atomes de carbone, par l'oxydation catalytique en phase gazeuse d'un alcénal, alcène, alcanol et/ou alcane, présentant le même nombre d'atomes de carbone, caractérisé en ce que l'on utilise, à titre de catalyseur, une masse suivant les revendications 1 à 7.
